# FASCICULE DE BREVET EUROPEEN

(11) **EP 3 468 671 B1**
(45) Date de publication et mention de la délivrance du brevet: **16.10.2024**
(21) Numéro de dépôt: 17745375.0
(22) Date de dépôt: 13.06.2017
(51) Int. Cl.: A61Q 1/00, A61Q 1/06, A61K 8/891, A61K 8/898

(54) **COMPOSITION COSMÉTIQUE COMPRENANT AU MOINS UN POLYMÈRE SILICONE-POLYURÉTHANE ET UNE RÉSINE DE SILICONE**
KOSMETISCHE ZUSAMMENSETZUNG MIT MINDESTENS EINEM SILIKON-POLYURETHAN-POLYMER UND EINEM SILIKONHARZ
COSMETIC COMPOSITION COMPRISING AT LEAST ONE SILICONE-POLYURETHANE POLYMER AND A SILICONE RESIN

(30) Priorité: 14.06.2016 FR 1655494
(43) Date de publication de la demande: 17.04.2019
(73) Titulaire: Chanel Parfums Beauté, 92200 Neuilly sur Seine (FR)
(72) Inventeur: SCHOLLER, Véronique, 93250 Villemomble (FR); MALVEZIN, Chantal, 60260 Lamorlaye (FR); RICHARD, Catherine, 92100 Boulogne Billancourt (FR); MASSON, Stéphane, 93500 Pantin (FR); DE CLERMONT GALLERANDE, Hélène, 94300 Vincennes (FR); CHAMPREDONDE, Elodie, 45000 Orleans (FR)
(74) Mandataire: Plasseraud IP
(86) Numéro de dépôt international: PCT/FR2017/051522
(87) Numéro de publication internationale: WO 2017/216475

(56) Documents cités:
- FR-A1- 2 926 022
- FR-A1- 2 939 033
- US-A1- 2003 235 552
- US-A1- 2008 305 068
- US-A1- 2010 266 648
- DATABASE GNPD [online] MINTEL; 25 April 2012 (2012-04-25), ANONYMOUS: "Plump & Stay Lip Colour", XP055694168, retrieved from www.gnpd.com Database accession no. 1776044
- ANONYMOUS: "Silmer UR-5050 Technical Data Sheet", 30 April 2016 (2016-04-30), XP002761202, Retrieved from the Internet <URL:http://www.siltech.com/tds/P5775.pdf> [retrieved on 20160826]

## Description

La présente invention a pour objet une composition cosmétique de maquillage ou de soin des matières kératiniques comprenant au moins un polymère silicone-polyuréthane et une résine de silicone. L'invention a également pour objet un procédé de maquillage ou de soin des matières kératiniques d'êtres humains comprenant l'application sur la peau de ladite composition.

Les compositions de maquillage ou de soin sont couramment employées pour apporter un aspect esthétique lors de l'application sur la peau et les lèvres, cet effet devant perdurer au cours du temps. Elles doivent notamment résister aux différents facteurs extérieurs susceptibles de modifier leur effet esthétique, comme la sueur ou la salive. En particulier, les produits cosmétiques, et notamment les rouges à lèvres, ne doivent pas migrer ou filer dans les rides ou ridules, ou transférer sur un tissu. Ils doivent également être agréables à appliquer et leur dépôt doit procurer une sensation de confort à l'utilisatrice, tout en conservant des propriétés esthétiques satisfaisantes.

Il est connu, afin de limiter le transfert de couleur des compositions cosmétiques et d'améliorer la tenue de leur teinte, d'incorporer dans les formules des polymères dits filmogènes. A titre d'exemples de polymères filmogènes usuellement mis en oeuvre, on peut citer les polyorganosiloxanes, notamment les polydiméthylsiloxanes (PDMS ou Dimethicone) tels que ceux décrits dans les documents US 6780402 (L'Oréal), US5318775 (Mary Kay Cosmetics), US4699780 (Estee Lauder); et US4578266 (Revlon) . Plus récemment, l'utilisation de polymères de type silicone-polyuréthane s'est développée. US 2008/0305068 décrit des compositions de maquillage présentant une bonne tenue et un effet "anti-transfert" et comprenant(i) au moins un polymère de type silicone-polyuréthane et(ii) au moins un élastomère qui peut être une gomme de silicone, une résine de silicone ou un polysiloxane fluide, préférablement une gomme de silicone.

Toutefois, ces polymères filmogènes ne permettent pas toujours d'obtenir de bons résultats en termes de tenue de la couleur du maquillage, et de résistance aux frottements.

Il serait donc souhaitable de disposer de compositions cosmétiques présentant de bonnes propriétés de tenue du maquillage, notamment de la couleur, tout en présentant des propriétés améliorées de résistance aux frottements.

Le but de la présente invention est donc de proposer une composition de maquillage ou de soin des matières kératiniques, en particulier des lèvres, présentant d'excellentes propriétés de tenue du maquillage, et notamment de sa couleur, et de résistance aux frottements.

L'invention a ainsi pour objet, selon un premier aspect, une composition cosmétique de maquillage ou de soin des matières kératiniques comprenant, dans un milieu physiologiquement acceptable, au moins un polymère silicone-polyuréthane et une résine de silicone, comme spécifié dans la revendication 1.

L'invention a également pour objet, selon un second aspect, un procédé de maquillage ou de soin des matières kératiniques, en particulier des lèvres, comprenant l'application sur lesdites matières kératiniques d'une telle composition.

Enfin, l'invention a pour objet, selon un troisième aspect, l'utilisation d'une résine siliconée particulière, pour améliorer la résistance aux frottements et la tenue d'une composition cosmétique comprenant, dans un milieu physiologiquement acceptable, au moins un polymère silicone-polyuréthane comme spécifié dans la revendication 15.

Il est en effet du mérite de la demanderesse d'avoir observé qu'il était possible de formuler des compositions présentant des propriétés de tenue et de résistance aux frottements améliorées en associant un polymère silicone-polyuréthane particulier avec une résine de silicone de type MQ, ledit polymère silicone-polyuréthane pouvant être utilisé en une teneur suffisamment faible pour ne pas altérer la résistance aux frottements de à la composition.

### Polymère de silicone-polyuréthane

La composition selon l'invention comprend au moins un polymère de silicone-polyuréthane. Le polymère de silicone-polyuréthane est le produit de réaction d'un polyorganosiloxane fonctionnalisé par des groupements hydroxyle, comprenant de préférence deux ou plus groupements hydroxyle, avec un composé diisocyanate. Le polyorganosiloxane fonctionnalisé par des groupements hydroxyle répond typiquement à la structure de formule I: 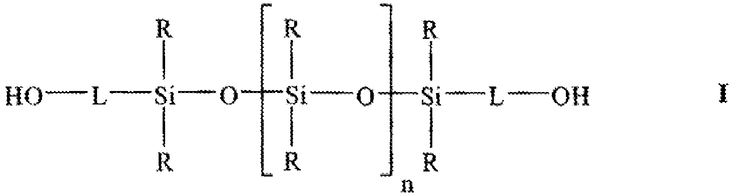
dans laquelle R est choisi indépendamment à chaque occurrence parmi un atome d'hydrogène, un groupe hydroxyle, et des groupes hydrocarbonés éventuellement substitués contenant de 1 à 10 atomes de carbone, et en particulier parmi un groupe alkyle, alcényle, alcynyle, aryle, aryle-alkyle ou alkyl-aryle substitués ou non ; de préférence, R est choisi parmi des groupes alkyle ou alcényle en C1-6 linéaires, cycliques ou ramifiés et optionnellement substitués, y compris, sans limitation, les groupes méthyle, éthyle, propyle, isopropyle, butyle, isobutyle, t-butyle, amyle, hexyle, cyclohexyle, vinyle, allyle ou aryle en C1-8, aryle-alkyle ou alkyl-aryle, y compris, sans limitation, phényle, benzyle, tolyle, xylyle ;
dans laquelle chacun des groupes R ci-dessus peut être substitué de façon facultative par un ou plusieurs hétéroatomes, y compris l'oxygène, l'azote, le phosphore et un halogène, en particulier fluor, comme illustré par les groupes fluoroalkyles (perfluoroalkyles) tels que les mono-, di- et tri fluorométhyle, perfluorophényle, et les groupes alkyle amino substitués en C1-6, y compris ceux répondant à la formule - (CH₂)₁₋₆-NR^{N}₂ et -(CH₂)₁₋₆-NR^{N}-(CH₂)₁₋₆-NR^{N}₂ où R^{N} est typiquement l'hydrogène, mais peut également être un groupe méthyle, éthyle, propyle, et analogues; des groupes polyéther, y compris, sans limitation, les groupes d'oxyde de polyéthylène répondant à la formule -(CH₂CH₂O)ₙ-, les groupes d'oxyde de propylène répondant à la formule -(CH(CH₃)CH₂O)ₙ- et des combinaisons de ceux-ci ; et des fonctions oxyde d'amine, phosphate, hydroxyle, ester et/ou carboxylate, ou analogues ; ou
dans laquelle R peut comprendre un groupe supplémentaire - L-0H;
dans laquelle L représente soit une liaison soit un groupe de liaison ; de préférence L est un groupe de liaison choisi parmi des hydrocarbures divalents ayant de 1 à 10 atomes de carbone, y compris un groupe alkyle, alcényle, alcynyle, aryle, alkyl-aryle ou aryl-alkyle divalents, comme par exemple un groupe alkyle en C1-10, y compris, sans limitation, les groupes bivalents de formule -(CH₂)₁₋₁₀-, de préférence -(CH₂)₁₋₆-, et plus préférablement, L est -CH₂CH_{2_}CH₂-;
et où n est un nombre entier entre 0 et 5000, de préférence entre 1 et 200, plus préférentiellement entre 10 et 100, et plus préférentiellement encore entre 10 et 50. De préférence, R représente au moins une ou plusieurs occurrences d'un groupe méthyle, plus préférentiellement, R représente un groupe méthyle à la totalité ou à la quasi-totalité des occurrences, ce qui signifie que R représente un groupe méthyle à plus de 90% des occurrences, notamment à plus de 95% des occurrences, voire à plus de 98% des occurrences.

Par exemple, le polyorganosiloxane fonctionnalisé par des groupements hydroxyle peut comprendre un polyméthylsiloxane répondant à la structure de formule Ia: 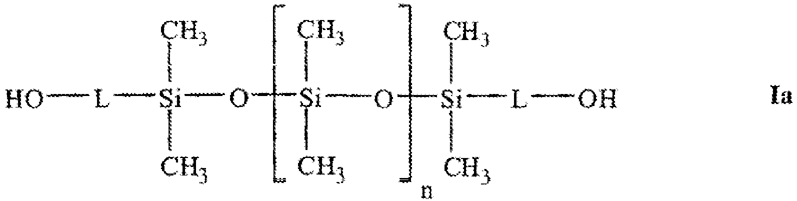 dans laquelle L et n sont tels que définis précédemment.

Selon l'invention, le polyorganosiloxane fonctionnalisé par des groupements hydroxyle comprend un polyméthylsiloxane répondant par exemple à la structure de formule Ib: 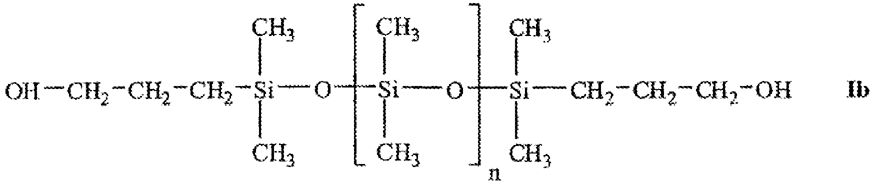 où n est tel que défini ci-dessus.

Le diisocyanate utilisé selon l'invention répond à la formule O=C=N-R¹-N=C=O, où R¹ est un groupe hydrocarboné divalent contenant de 1 à 20 atomes de carbone, y compris optionnellement substitué par un ou plusieurs hétéroatomes, et en particulier R¹ peut être choisi parmi un groupe alkyle cyclique, alcényle, alcynyle, aryle, alkyl-aryle ou aryl-alkyle linéaire, cyclique ou ramifié, facultativement substitué, y compris, sans limitation:
i. un groupe de formule : 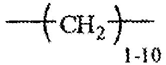
ii. un groupe de formule : 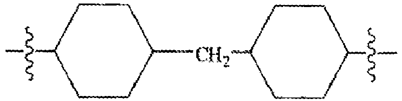
iii. un groupe de formule : 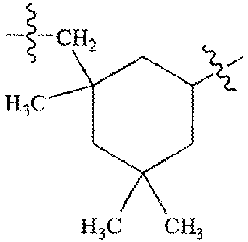
iv. un groupe de formule : 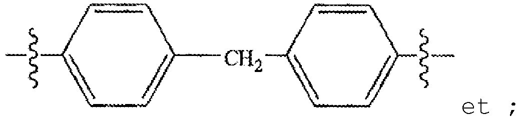
v. un groupe de formule : 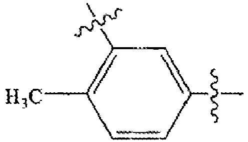 et des combinaisons de ceux-ci.

Les diisocyanates pouvant convenir dans le cadre de l'invention comprennent, de manière non limitative, le diisocyanate de toluène; le méthylène diphényle diisocyanate, y compris le 2,2'-MDI, 2,4'-MDI et 4,4'-MDI; le diisocyanate de 1,6-hexaméthylène; le diisocyanate d'isophorone; le diisocyanate de méthylène dicyclohexyle; le diisocyanate de xylène; le diisocyanate de cyclohexane; le diisocyanate de 3,3'-diméthyl-4,4'-diphénylméthane; le diisocyanate de p-phénylène; le diisocyanate de m-phénylène; l'isocyanate de 4,4'-isopropylidène dicyclohexyle; et leur équivalents.

Dans un mode de réalisation préféré, le diisocyanate est choisi dans le groupe constitué par le diisocyanate de 1,6-hexaméthylène, le diisocyanate de dicyclohexyle méthylène, le diisocyanate d'isophorone, et leurs combinaisons. Dans un mode de réalisation, le diisocyanate comprend ou consiste essentiellement en du diisocyanate de 1,6-hexaméthylène. Dans un autre mode de réalisation, le diisocyanate comprend ou consiste essentiellement en du diisocyanate d'isophorone. Dans encore un autre mode de réalisation, le diisocyanate comprend ou consiste essentiellement en du diisocyanate de dicyclohexyle méthylène, ce mode de réalisation étant particulièrement préféré.

Selon un mode préféré de réalisation, le polymère de silicone-polyuréthane selon l'invention comprend des unités récurrentes dérivées du polyorganosiloxane fonctionnalisé par des groupements hydroxyle et du diisocyanate sous la forme d'un copolymère alterné AB, où l'unité A a la structure de formule II: 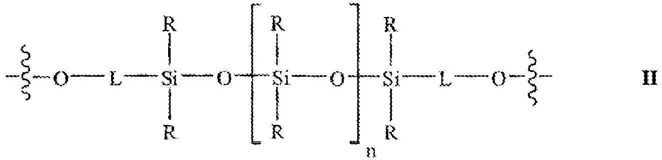
dans laquelle R, L et n sont définis comme précédemment en relation avec la formule Ib, et où l'unité B a la structure de formule III: 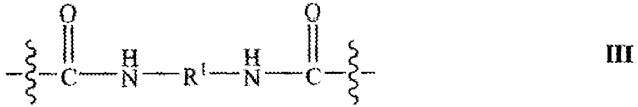
où R¹ est tel que défini précédemment, et dans lequel les unités A et B sont arrangées selon une configuration linéaire, ramifiée ou cyclique, et de préférence linéaire.

Dans un mode de réalisation préféré, le polymère de silicone-polyuréthane mis en oeuvre dans les compositions cosmétiques de l'invention est un polymère linéaire comprenant le produit de réaction de formule Ib avec un diisocyanate choisi dans le groupe constitué par le diisocyanate de 1,6-hexaméthylène, le diisocyanate de dicyclohexyle méthylène, le diisocyanate d'isophorone, et leurs combinaisons.

Un polymère de silicone-polyuréthane est par exemple disponible auprès Siltech Corporation sous la forme d'une prémélange dans l'isododécane, sous la référence commerciale SILMER UR 5050 ou UR 100100.

La composition selon l'invention comprend notamment le polymère de silicone-polyuréthane en une teneur allant de 8 à 16 % en poids de matière active de polymère, par rapport au poids total de la composition, de préférence de 10 à 15 % en poids.

Il est en effet du mérite de la demanderesse d'avoir observé que des compositions comprenant plus de 16% en matière active de silicone-polyuréthane présentaient une résistance aux frottements et une tenue moins bonnes.

### Résine de silicone

La composition selon l'invention, comprend au moins une résine de silicone de type MQ.

De manière générale, par le terme « résine », on entend un composé dont la structure est tridimensionnelle. Ainsi, au sens de la présente invention, une polydiméthylsiloxane n'est pas une résine de silicone.

La nomenclature des résines de silicone (également appelées résines de siloxanes ou résine siliconées) est connue sous le nom de "MDTQ", la résine étant décrite en fonction des différentes unités monomériques siloxane qu'elle comprend, chacune des lettres "MDTQ" caractérisant un type d'unité.

La lettre « M » représente l'unité Monofonctionnelle de formule R1R2R3S1O_{1/2}, l'atome de silicium étant relié à un seul atome d'oxygène dans le polymère comprenant cette unité.

La lettre « D » signifie une unité Difonctionnelle R1R2S1O_{2/2} dans laquelle l'atome de silicium est relié à deux atomes d'oxygène.

La lettre « T » représente une unité Trifonctionnelle de formule RlSiO_{3/2}.

De telles résines sont décrites par exemple les demandes de brevets US 2,676,182, US 3,627,851, US 3,772,247, US 5,248,739 ou encore US 5,082,706, US 5,319,040, US 5,302, 685 et US 4,935,484.

Dans les motifs M, D, T définis précédemment, R, à savoir Rl et R2, représente un radical hydrocarboné (notamment alkyle) ayant de 1 à 10 atomes de carbone, un groupe phényl, un groupe phénylalkyl ou bien encore un groupe hydroxyle.

Enfin, la lettre « Q » signifie une unité tétrafonctionnelle SiO_{4/2} dans laquelle l'atome de silicium est lié à quatre atomes d'oxygènes eux-mêmes liés au reste du polymère.

Diverses résines siliconées de propriétés différentes peuvent être obtenues à partir de ces différentes unités, les propriétés de ces polymères variant en fonction du type de monomères (ou unités), de la nature et du nombre du radical R, de la longueur de la chaîne polymérique, du degré de ramification et de la taille des chaînes pendantes.

Les résines siliconées utilisées dans les compositions selon l'invention sont des résines siliconées de type MQ. Selon un mode préféré de réalisation, les résines MQ se présentent sous forme solide, notamment sous forme de poudre.

Selon un mode préféré de réalisation, les résines siliconées utilisées dans les compositions selon l'invention sont filmogènes, c'est-à-dire qu'elles permettent de former un film sur les matières kératiniques sur lesquelles elles sont appliquées. Cela exclue notamment les résines de types polyméthylsilsesquioxane (ou résines T) qui se présentent sous forme de poudre insoluble et ne sont pas filmogènes.

Selon un autre mode préféré de réalisation, les résines siliconées utilisées dans les compositions selon l'invention sont solubles à chaud dans les solvants siliconés, par opposition aux résines de types polyméthylsilsesquioxane (ou résines T) qui se présentent sous forme de poudre insoluble.

Selon l'invention on utilise une résine MQ. A titre d'exemple de résines siliconées de type MQ, on peut citer les alkylsiloxysilicates de formule [(Rl)₃Si_{1/2}]ₓ(SiO_{4/2})_{y} (unités MQ) dans laquelle x et y sont des entiers allant de 50 à 80, et tel que le groupement Rl représente un radical tel que défini précédemment, et de préférence est un groupement alkyle ayant de 1 à 8 atomes de carbone ou un groupe hydroxyle, de préférence, un groupe méthyl. Les résines MQ de type triméthylsiloxysilicate sont ainsi préférées.

Comme exemple de résines siliconées solides de type MQ de type triméthylsiloxysilicate on peut citer celles commercialisées sous la référence SR1000 par la société General Electric, sous la référence TMS 803 par la société Wacker, sous la dénomination "KF-7312J" par la société Shin-Etsu, "DC 749", "DC 593" par la société Dow Corning.

Comme résines siliconées comprenant des motifs MQ siloxysilicates, on peut également citer les résines phénylalkylesiloxysilicate, telle que la phénylpropyldiméthylsiloxysilicate (Silshine 151 commercialisée par la société General Electric). La préparation de telles résines est décrite notamment dans le brevet US5817302.

A titre d'exemple de résines siliconées de type T, on peut citer les polysilsesquioxanes de formule (RSiO_{3/2})ₓ (unités T) dans laquelle x est supérieur à 100 et tel que le groupement R est un groupement alkyle ayant de 1 à 10 atomes de carbone, lesdites polysilsesquioxanes pouvant en outre comprendre des groupes terminaux Si-OH. Les résines polyméthylsilsesquioxanes dans lesquelles R représente un groupe méthyl, sont par exemple celles commercialisées :
- par la société Wacker sous la référence Resin MK tels que la Belsil PMS MK : polymère comprenant des unités répétitives CH₃SiO_{3/2} (unités T), pouvant aussi comprendre jusqu'à 1% en poids d'unités (CH₃)₂SiO_{2/2} (unités D) et présentant un poids moléculaire moyen d'environ 10000 g/mol, ou
- par la société SHIN-ETSU sous les références KR-220L qui sont composées 11 d'unités T de formule CH₃SiO_{3/2} et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88% d'unités T et 12% d'unités diméthyl D et ont des groupes terminaux Si-OH.

A titre de résine comprenant des motifs MQT, on connaît notamment celles citées dans le document US 5 110 890. Une forme préférée de résines de type MQT sont les résines MQT-propyl (également appelée MQTPr). De telles résines notamment celles décrites et préparées dans la demande WO 2005/075542. La résine MQ-T-propyl comprend de préférence les unités :
(i) (R1₃SiO_{1/2})ₐ
(ii) (R2₂SiO_{1/2})_{b}
(iii) (R3SiO_{1/2})_{c} et
(iv) (SiO_{4/2})_{d}

avec R1, R2 et R3 représentant indépendamment un radical hydrocarboné (notamment alkyle) ayant de 1 à 10 atomes de carbone, un groupe phényl, un groupe phénylalkyl ou bien encore un groupe hydroxyle et de préférence un radical alkyle ayant de 1 à 8 atomes de carbone ou un groupement phényl,
a étant compris entre 0,05 et 0,5,
b étant compris entre zéro et 0,3,
c étant supérieur à zéro,
d étant compris entre 0,05 et 0,6, a + b + c + d = 1, et
a, b, c et d étant des fractions molaires, à condition que plus de 40 % en moles des groupements R3 de la résine de siloxane soient des groupements propyle.

Les résines de siloxane peuvent être obtenues par un procédé comprenant la réaction de
A) une résine MQ comprenant au moins 80 % en moles d'unités (R1₃SiO_{1/2})ₐ et (SiO_{4/2})_{d}, Rl représentant un groupement alkyle ayant de 1 à 8 atomes de carbone, un groupement aryle, un groupement carbinol ou un groupement amino, a et d étant supérieurs à zéro, le rapport a/d étant compris entre 0,5 et 1,5 ; et de
B) une résine de propyle T comprenant au moins 80 % en moles d'unités (R3SiO_{1/2})_{c}, R3 représentant un groupement alkyle ayant de 1 à 8 atomes de carbone, un groupement aryle, un groupement carbinol ou un groupement amino, c étant supérieur à zéro, à condition qu'au moins 40 % en moles des groupements R3 soient des groupements propyle, où le ratio massique A/B est compris entre 95:5 et 15:85, de préférence le ratio massique A/B est de 30:70.

La composition selon l'invention comprend une résine de silicone MQ, notamment en une teneur allant de 1 à 20 % en poids de résine, par rapport au poids total de la composition, de préférence de 5 à 10 % en poids.

### Milieu physiologiquement acceptable

La composition selon l'invention comprend un milieu physiologiquement acceptable et de préférence cosmétiquement acceptable, c'est-à-dire qui ne présente pas d'effets secondaires délétères et en particulier qui ne produit pas de rougeurs, d'échauffements, de tiraillements ou de picotements inacceptables pour un utilisateur de produits cosmétiques.

### Huiles

Le milieu physiologiquement acceptable peut notamment comprendre au moins une huile.

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

L'huile peut être volatile ou non volatile, polaire ou apolaire. L'homme du métier veillera à choisir les huiles constituant le milieu physiologiquement acceptable de la composition selon l'invention de manière à ce que celles-ci soient compatibles avec le polymère silicone acrylate et la résine de silicone qu'elle contient.

Comme huiles pouvant être utilisées dans les compositions selon l'invention, on peut notamment citer les huiles hydrocarbonnées, les huiles de silicone, les huiles fluorosiliconées, les huiles fluorées, ainsi que leurs mélanges.

Parmi ces huiles, on préfère que la composition selon l'invention comprenne au moins une huile hydrocarbonée.

On entend par "huile hydrocarbonée", une huile contenant uniquement des atomes d'hydrogène et de carbone.

L'huile hydrocarbonée peut être volatile, et notamment présenter un point éclair allant de 40°C à 102°C, de préférence allant de 40°C à 55°C, et préférentiellement allant de 40°C à 50°C. L'huile volatile hydrocarbonée peut notamment être choisie parmi les huiles volatiles hydrocarbonées ayant de 8 à 16 atomes de carbone et leurs mélanges, et notamment :
- les alcanes ramifiés en C₈-C₁₆ comme les iso-alcanes (appelées aussi isoparaffines) en C₈-C₁₆, l'isododécane, l'isodécane, l'isohexadécane, et par exemple les huiles vendues sous les noms commerciaux d'Isopars ou de Permetyls,
- les alcanes linéaires, par exemple tels que le n-dodécane (C12) et le ntétradécane (C14) vendus par Sasol respectivement sous les références PARAFOL 12-97 et PARAFOL 14-97, ainsi que leurs mélanges, le mélange undécane-tridécane (Cétiol UT), les mélanges de n-undécane (Cll) et de n-tridécane (C13) obtenus aux exemples 1 et 2 de la demande WO2008/155059 de la Société Cognis et leurs mélanges.

L'huile hydrocarbonée volatile est de préférence l'isododécane.

L'huile hydrocarbonée peut être non volatile hydrocarbonée, de préférence polaire.

En particulier, ladite huile non volatile peut être une huile ester, en particulier ayant entre 18 et 70 atomes de carbones.

A titre d'exemples, on peut citer les mono-, di- ou triesters.

Les huiles esters peuvent notamment être hydroxylées.

L'huile ester non volatile peut de préférence être choisie parmi :
- les monoesters comprenant entre 18 et 40 atomes de carbone au total, en particulier les monoesters de formule R₁COOR₂ dans laquelle R1 représente le reste d'un acide gras linéaire ou ramifié comportant de 4 à 40 atomes de carbone et R2 représente une chaîne hydrocarbonée notamment ramifiée contenant de 4 à 40 atomes de carbone à condition que R1 + R2 soit 18, comme par exemple l'huile de Purcellin (octanoate de cétostéaryle), l'isononanoate d'isononyle, le benzoate d'alcool en C12 à C15, le palmitate d'éthyl 2-hexyle, le néopentanoate d'octyledodécyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle. De façon préférée, il s'agit des esters de formule R₁COOR₂ dans laquelle R1 représente le reste d'un acide gras linéaire ou ramifié comportant de 4 à 40 atomes de carbone et R2 représente une chaîne hydrocarbonée notamment ramifiée contenant de 4 à 40 atomes de carbone, R1 et R2 étant tels que R1 + R2 soit 18. De façon préférée, l'ester comprend entre 18 et 40 atomes de carbone au total. A titre de monoesters préférés, on peut citer l'isononanoate d'isononyle.
- les diesters, notamment comprenant entre 18 et 60 atomes de carbone au total, en particulier entre 18 et 50 atomes de carbone au total. On peut notamment utiliser les diesters de diacide carboxylique et de monoalcools, tel que de préférence le diisostéaryle malate. Alternativement, les diesters peuvent être les diesters de glycol et de monoacides carboxyliques, tels que le diheptanoate de néopentylglycol ou le polyglycéryle-2 diisostéarate (notamment tel que le composé vendu sous la référence commerciale DERMOL DGDIS par la société Alzo)
- les triesters, notamment comprenant entre 35 et 70 atomes de carbone au total, en particulier tel que les triesters de triacide carboxylique, tels que le triisostéaryle citrate, ou le tridécyl trimellitate, ou les triesters de glycol et de monoacides carboxyliques tel que le triisostéarate de polyglycérol-2 ;
- les tétraesters, notamment ayant un nombre total de carbone allant de 35 à 70, tel que les tétraesters de penthaerythritol ou de polyglycerol et d'un monoacide carboxylique, par exemple tels que le tétrapélargonate de pentaérythrityle, le pentaerythrityle de tetraisostéarate, le tétraisononanoate de pentaérythrityle, le tri décyl-2 tétradécanoate de glycéryle, le tétraisostéarate de polyglycéryle-2 ou encore le tétra décyl-2 tétradécanoate de pentaérythrityle
- les polyesters obtenus par condensation de dimère et/ou trimère d'acide gras insaturé et de diol tels que ceux décrits dans la demande de brevet FR 0 853 634, tels qu'en particulier de l'acide dilinoléique et du 1,4- butanediol.
- les esters et polyesters de dimère diol et d'acide mono-ou dicarboxylique, tels que les esters de dimère diol et d'acide gras et les esters de dimère diols et de dimère diacide carboxylique, en particulier pouvant être obtenus à partir d'un dimère diacide carboxylique dérivé en particulier de la dimérisation d'un acide gras insaturé notamment en C8 à C34, notamment en C12 à C22, en particulier en C16 à C20, et plus particulièrement en C18, tels que les esters de diacides dilinoléiques et de dimères diols dilinoléiques, par exemple tels que ceux commercialisés par la société NIPPON FINE CHEMICAL sous la dénomination commerciale LUSPLAN DD-DA5^{®} et DD-DA7^{®}
- les copolymères vinylpyrrolidone/1-héxadécéne, comme par exemple celui vendu sous la dénomination ANTARON V-216 (également appelé Ganex V216) par la société ISP (MW=7300 g/mol), - les huiles hydrocarbonées végétales telles que les triglycérides d'acides gras (liquides à température ambiante), notamment d'acides gras ayant de 7 à 40 atomes de carbone, tels que les triglycérides des acides heptanoïque ou octanoïque ou l'huile de jojoba, en particulier, on peut citer les triglycérides saturés tels que le caprylic/capric triglycéride, le triheptanoate de glycéryle, le trioctanoate de glycérine, les triglycérides d'acide en C18_36 tels que ceux commercialisés sous la référence DUB TGI 24 commercialisé par Stéarineries Dubois), et les triglycérides insaturés tels que l'huile de ricin, l'huile d'olive, l'huile de ximénia, l'huile de pracaxi ;
- et leurs mélanges.

On entend par "huile de silicone", une huile comprenant au moins un atome de silicium, et notamment au moins un groupe Si-O. L'huile de silicone peut être volatile ou non volatile.

Comme huile de silicone non volatile, on peut notamment citer les polydiméthylsiloxanes renfermant au moins 8 atomes de silicium, les polyalkylméthylsiloxane dont la chaîne alkyle renferme de 8 à 20 atomes de carbone et les huiles identifiées par le nom INCI phenyl trimethicone.

Comme huile de silicone volatile, on peut citer notamment certaines diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, les composés identifiés par les noms INCI methyl trimethicone et caprylyl methicone et leurs mélanges.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camellia.

On entend par « huiles fluorées », une huile contenant au moins un atome de fluor, telle que le nonafluorométhoxybutane ou le perfluorométhyl-cyclopentane, le perfluorodiméthylcyclohexane, le perfluoroperhydrophénanthrène, la perfluorodécaline, et leurs mélanges, sans que cette liste ne soit limitative.

La composition selon l'invention comprend de préférence de 40 à 80% en poids d'huile, notamment hydrocarbonée, en particulier de 45 à 75% en poids, et plus préférentiellement de 50 à 70% en poids d'huile.

### Cire et gélifiant lipophile

La composition selon l'invention peut en outre comprendre au moins une cire et/ou au moins un gélifiant lipophile.

Par « cire », on entend un corps gras ayant une température de fusion supérieure à 30°C et généralement inférieure à 100°C, qui est liquide dans les conditions de préparation de la composition et présente à l'état solide une organisation cristalline anisotrope. Des exemples de cires sont notamment les cires végétales, minérales ou synthétiques, ces dernières pouvant avantageusement être des cires hydrocarbonées ou siliconées. On peut ainsi mentionner les cires de Carnauba, de Candelilla, de riz, d'abeille (Cera alba), de polyéthylène éventuellement fonctionnalisées, et de paraffine, ainsi que l'ozokérite, les cires microcristallines, les alcools gras linéaires en C₁₄₋C₂₂ et les triesters d'acides en C₈-C₂₀ et de glycérine tels que le tribéhénate de glycérine, et leurs mélanges, sans que cette liste ne soit limitative. On peut également citer le stéarate de glycol acétylé commercialisé par la société VEVY sous la dénomination commerciale CETACENE^{®}.

Selon un mode préféré de réalisation, la composition selon l'invention comprend au moins une cire apolaire. On entend par cire apolaire une cire apolaire hydrocarbonée ou siliconée.

Selon un mode de réalisation, la cire hydrocarbonée apolaire contient au moins 95% en poids de composés chimiques constitués de carbone et d'hydrogène. Ces composés chimiques sont avantageusement choisis parmi les alcanes saturés, linéaires ou ramifiés. En particulier, la cire apolaire peut être choisie parmi les cires hydrocarbonées linéaires. Les cires hydrocarbonées linéaires incluent les polymères et copolymères de l'éthylène, les cires de paraffine linéaires et les cires de Fischer Tropsch.

La cire apolaire peut alternativement être une cire de type silicone polyoxyalkylénée c'est-à-dire une silicone comportant au moins un groupement oxyalkyléné de type (-CxH₂ₓO)a dans lequel x peut varier de 2 à 6 et a est supérieur à ou égal à 2.

Selon un mode préféré de réalisation, la composition selon l'invention comprend au moins une cire de polyéthylène.

La composition selon l'invention peut notamment comprendre une cire en une teneur allant de 1 à 20% en poids, de préférence de 5 à 15% en poids, par rapport au poids total de la composition.

Des exemples de gélifiants lipophiles sont notamment des charges permettant de modifier la rhéologie ou la texture de la composition.

Par « charge », il faut comprendre les particules incolores ou blanches, minérales ou de synthèse, solides de toutes formes, qui se présentent sous une forme insoluble et dispersée dans le milieu de la composition, quelle que soit la température à laquelle la composition est fabriquée. Les charges peuvent être minérales ou organiques de toute forme, plaquettaires, sphériques ou oblongues, quelle que soit la forme cristallographique (par exemple feuillet, cubique, hexagonale, orthorombique, etc).

En particulier, la charge peut être choisie parmi le talc, le mica, la silice, le kaolin, une argile type hectorite (la bentone), les particules de silice pyrogénée, éventuellement traitées hydrophile ou hydrophobe, les poudres de polyamide (Nylon^{®}) (Orgasol^{®} de chez Atochem), de poly-(3-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning), les microbilles de résine de silicone (Tospearls^{®} de TOSHIBA, par exemple), le carbonate de calcium précipité, le carbonate et l'hydro-carbonate de magnésium, l'hydroxyapatite, les microsphères de silice creuses (Silica Beads^{®} de Maprecos), les particules de polyorganosiloxanes élastomères, les savons métalliques dérivés d'acides organiques carboxyliques ayant de 8 à 22 atomes de carbone, de préférence de 12 à 18 atomes de carbone, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélange. De façon préférée, la charge est choisie parmi la silice, le kaolin, la bentone, l'amidon, la lauroyl-lysine, les particules de silice pyrogénée, éventuellement traitées hydrophile ou hydrophobe, et leurs mélanges. Selon un mode préféré de réalisation, le gélifiant lipophile est de préférence la bentone.

La composition mise en oeuvre selon l'invention peut comprendre un ou plusieurs gélifiants lipophiles en une teneur allant de 0,1 à 13% en poids par rapport au poids total de la composition, en particulier de 0,2 à 10% en poids par rapport au poids total de la composition.

### Matières colorantes

La composition selon l'invention peut comprendre, en outre, au moins une matière colorante qui peut être choisie parmi les pigments, les nacres, les colorants liposolubles, les laques (pigment organique) et leurs mélanges.

Par pigments, il faut comprendre des particules blanches ou colorées, minérales ou organiques, insolubles dans une solution aqueuse, destinées à colorer et/ou opacifier la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les pigments utilisés dans la composition cosmétique selon l'invention peuvent être traités en surface par un agent de traitement hydrophobe.

L'agent de traitement hydrophobe peut être choisi parmi les silicones comme les méthicones, les diméthicones, les perfluoroalkylsilanes, les alkyl alcoxy silanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Selon un mode préféré de réalisation, l'agent de traitement hydrophobe est choisi parmi les alkyl alcoxy silanes, en particulier l'octyl triéthoxy silane (OTS).

Par " nacres ", il faut comprendre des particules colorées de toute forme, irisées ou non, notamment produites par certains mollusques dans leur coquille ou bien synthétisées et qui présentent un effet de couleur par interférence optique.

Par " colorants ", il faut comprendre des composés généralement organiques solubles dans les corps gras comme les huiles.

Les colorants liposolubles sont par exemple le rouge Soudan, le DC Red 17, le DC Green 6, le β-carotène, le brun Soudan, le DC Yellow 11, le DC Violet 2, le DC orange 5, le jaune quinoléine.

Les matières colorantes peuvent être présentes en une teneur allant de 0,1 à 15 % en poids, notamment de 1 à 10 % en poids, et en particulier de 1 à 10 % en poids, par rapport au poids total de la composition cosmétique.

### Actifs

La composition selon l'invention peut par ailleurs contenir un ou plusieurs actifs, en particulier choisi parmi les agents hydratants (notamment la vitamine E), les agents cicatrisants et/ou les agents anti-âges, de la peau et/ou des lèvres, et en particulier des lèvres.

Selon ce mode de réalisation, l'invention concerne également un procédé de soin et/ou de maquillage (non thérapeutique) de la peau et/ou des lèvres, et en particulier des lèvres comprenant l'application d'une composition selon l'invention sur la peau et ou les lèvres.

Le dépôt réalisé avec une composition selon l'invention présentant un bon niveau de tenue, permet d'assurer la rémanence de l'actif sur la peau et/ou les lèvres et d'améliorer ainsi l'efficacité du soin (effet hydratant, cicatrisant et/ou ati-âge) de la peau et/ou des lèvres.

La composition utilisée selon l'invention peut en outre renfermer des agents humectants tels que l'acide hyaluronique et ses sels et/ou les polyols tels que la glycérine.

### Ingrédients cosmétiques usuels additionnels

La composition selon l'invention peut également comprendre tout ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les séquestrants, et leurs mélanges.

En particulier, la composition selon l'invention peut comprendre au moins un filtre solaire.

### Galénique

La composition selon l'invention peut être avantageusement utilisée pour le soin ou le maquillage de la peau ou des lèvres, et en particulier le maquillage des lèvres.

En particulier, le produit de l'invention peut se présenter sous forme d'une couche de base dans le cadre d'un produit de maquillage double-couche de la peau ou des lèvres.

### Procédé de maquillage

L'invention a également pour objet une procédé de maquillage ou de soin des matières kératiniques, en particulier des lèvres, comprenant l'application sur lesdites matières kératiniques d'au moins une composition telle que définie précédemment.

Selon un mode préféré de réalisation, le procédé selon l'invention comprend :
- l'application sur lesdites matières kératiniques d'au moins une composition telle que définie précédemment, en tant que composition de base (aussi appelée « base coat »), et
- l'application sur ladite composition de base, d'une composition de revêtement (aussi appelée « top coat »).

La composition de revêtement « top coat » est notamment choisie par l'homme du métier de manière à être incompatible avec la composition de base afin d'éviter la migration des constituants de chaque composition vers l'autre, ce qui altérerait la tenue et l'intensité de la couleur conférée par la composition de base selon l'invention, et atténuerait la brillance conférée par la composition de revêtement « top coat ».

La composition de revêtement (« top coat ») comprend, de préférence, au moins une huile non volatile, de préférence brillante, incompatible avec la composition de base. L'huile non volatile peut notamment être une huile de silicone non volatile.

La composition de revêtement (« top coat ») permet de conférer de la brillance à la composition de base, et de diminuer voire de faire disparaître la sensation de collant procurée par la composition de base lorsqu'elle est appliquée seule.

### Utilisation

L'invention a encore pour objet, selon un autre aspect, l'utilisation d'une résine siliconée particulière, pour améliorer la résistance aux frottements et la tenue d'une composition cosmétique comprenant, dans un milieu physiologiquement acceptable, au moins un polymère silicone-polyuréthane selon la revendication 15.

Les exemples suivants sont donnés à titre illustratif.

Les pourcentages sont des pourcentages en poids.

### Exemples

On a préparé deux formules liquides pour les lèvres ayant les compositions suivantes :

| | Composition 1 selon l'invention (% en poids) | Composition 2 comparative (% en poids) |
|---|---|---|
| Résine de silicone MQ (Belsil TMS 803 de Wacker) | 7,0 | - |
| Polymère silicone-polyuréthane à 40% dans l'isododécane (SILMER UR-5050 de Siltech) | 30,0 | 30,0 |
| Isododécane | 27,0 | 34,0 |
| Cire de polyéthylène | 10,0 | 10,0 |
| Hectorite (Bentone Gel) | 10,0 | 10,0 |
| Mica | 7,5 | 7,5 |
| Matières colorantes | 6,0 | 6,0 |
| Diisostéaryl malate | 1,5 | 1,5 |
| Vitamine E | 0,5 | 0,5 |
| Conservateurs | 0,5 | 0,5 |

Les formules ont été appliquées (3 couches successives) sur les lèvres d'un panel de 9 personnes.

Les propriétés des compositions ont été évaluées visuellement par une maquilleuse expérimentée sur les critères suivants :
- tenue de la couleur des compositions après un repas (pâtes en sauce),
- résistance au frottement (essuyage des compositions avec un mouchoir).

La maquilleuse a ainsi observé que la composition 1 selon l'invention laissait davantage de dépôt résiduel sur les lèvres après essuyage avec un mouchoir que la composition 2 comparative.

En outre, le démaquillage de la composition 1 s'est montré beaucoup plus difficile que celui de la composition 2 comparative car la composition 1 selon l'invention adhère davantage à la muqueuse (meilleure tenue du maquillage).

Dans un autre essai, les formules des compositions 1 et 2 ont été, de nouveau, appliquées (3 couches successives) sur les lèvres d'un panel de 9 personnes, puis, cette fois, recouvertes d'un « top coat » brillant incolore présentant la formule suivante :

| | Top coat incolore (% en poids) |
|---|---|
| Huile de silicone non volatile Viscosité 1 000 000 cs | 25,0 |
| Huile de silicone non volatile Viscosité 350cs | 75,0 |

Le résultat maquillage a été évalué visuellement par une maquilleuse expérimentée sur les critères suivants :
- teinte,
- homogénéité,
- netteté des contours,
- brillance.

La maquilleuse a ainsi observé que la composition 1 selon l'invention permettait d'obtenir une teinte plus intense (notamment au cours de la journée), homogène lors de l'application et filant moins (contours plus nets) que la composition 2 comparative.

Un test de tenu des compositions après un repas (pâtes en sauce) a également permis de montrer une meilleure tenue de la teinte obtenue avec la composition 1 selon l'invention par rapport à la composition 2 comparative.

## Revendications

1. Composition cosmétique de maquillage ou de soin des matières kératiniques comprenant, dans un milieu physiologiquement acceptable, au moins un polymère silicone-polyuréthane et une résine de silicone de type MQ,
ledit polymère silicone polyuréthane étant le produit de réaction d'un polyorganosiloxane fonctionnalisé par des groupements hydroxyle répondant à la structure de formule lb: 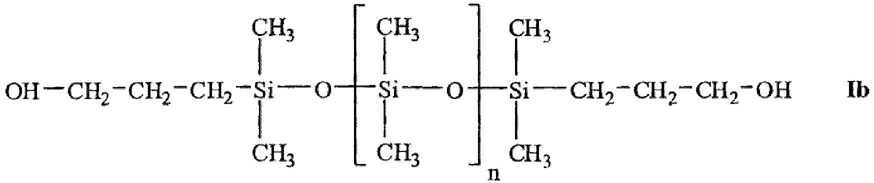
dans laquelle n est un nombre entier entre 0 et 5000, de préférence entre 1 et 200, plus préférentiellement entre 10 et 100, et plus préférentiellement encore entre 10 et 50,
avec un composé diisocyanate répondant à la formule O=C=N-R¹-N=C=O, où R¹ est un groupe hydrocarboné divalent contenant de 1 à 20 atomes de carbone, et en particulier R¹ est un groupe de formule : 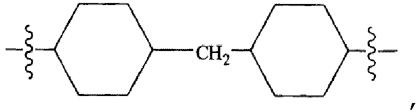
ladite résine de silicone MQ se présentant de préférence sous forme solide, notamment sous forme de poudre.

2. Composition selon la revendication précédente, **caractérisée en ce que** le composé diisocyanate est choisi dans le groupe constitué par le diisocyanate de 1,6-hexaméthylène, le diisocyanate de dicyclohexyle méthylène, le diisocyanate d'isophorone, et leurs combinaisons.

3. Composition selon l'une quelconque des revendications précédentes, **caractérisée en ce que** le polymère de silicone-polyuréthane est présent en une teneur allant de 8 à 16 % en poids de matière active de polymère, par rapport au poids total de la composition, de préférence de 10 à 15 % en poids.

4. Composition selon l'une quelconque des revendications précédentes, **caractérisée en ce que** la résine siliconée est une résine de type MQ alkylsiloxysilicates de formule [(Rl)₃Si_{1/2}]ₓ(SiO_{4/2})_{y} dans laquelle x et y sont des entiers allant de 50 à 80, et le groupement Rl représente un radical hydrocarboné ayant de 1 à 10 atomes de carbone, un groupe phényl, un groupe phénylalkyl ou bien encore un groupe hydroxyle.

5. Composition selon la revendication précédente, **caractérisée en ce que** la résine MQ de type triméthylsiloxysilicate.

6. Composition selon l'une quelconque des revendications précédentes, **caractérisée en ce que** la résine de silicone MQ, est présente en une teneur allant de 1 à 20 % en poids de résine, par rapport au poids total de la composition, de préférence de 5 à 10 % en poids.

7. Composition selon l'une quelconque des revendications précédentes, **caractérisée en ce que** le milieu physiologiquement acceptable comprend au moins une huile hydrocarbonée volatile ou non volatile.

8. Composition selon la revendication précédente, **caractérisée en ce que** l'huile hydrocarbonée volatile comprend de 8 à 16 atomes de carbone, et est de préférence l'isododécane.

9. Composition selon l'une quelconque des revendications précédentes, **caractérisée en ce qu'**elle comprend au moins une cire et/ou au moins un gélifiant lipophile.

10. Composition selon la revendication précédente, **caractérisée en ce que** la cire est une cire de polyéthylène et le gélifiant lipophile est une argile.

11. Composition selon l'une quelconque des revendications précédentes, **caractérisée en ce qu'**elle comprend au moins une matière colorante choisie parmi les pigments, les nacres, les colorants liposolubles, les laques (pigment organique) et leurs mélanges.

12. Procédé de maquillage ou de soin des matières kératiniques comprenant l'application sur lesdites matières kératiniques d'au moins une composition selon l'une des revendications 1 à 11.

13. Procédé de maquillage ou de soin des matières kératiniques comprenant :
- l'application sur lesdites matières kératiniques d'au moins une composition selon l'une des revendications 1 à 11, en tant que composition de base, et
- l'application sur ladite composition de base, d'une composition de revêtement.

14. Procédé selon la revendication 13, **caractérisé en ce que** la composition de revêtement est incompatible avec la composition de base.

15. Utilisation d'une résine siliconée MQ de type triméthylsiloxysilicate, pour améliorer la résistance aux frottements et la tenue d'une composition cosmétique comprenant, dans un milieu physiologiquement acceptable, au moins un polymère silicone-polyuréthane, ledit polymère silicone polyuréthane étant le produit de réaction d'un polyorganosiloxane fonctionnalisé par des groupements hydroxyle répondant à la structure de formule lb: 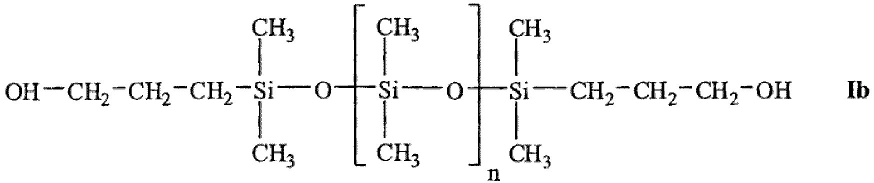
dans laquelle n est un nombre entier entre 0 et 5000, de préférence entre 1 et 200, plus préférentiellement entre 10 et 100, et plus préférentiellement encore entre 10 et 50,
avec un composé diisocyanate répondant à la formule O=C=N-R¹-N=C=O, où R¹ est un groupe hydrocarboné divalent contenant de 1 à 20 atomes de carbone, et en particulier R¹ est un groupe de formule :

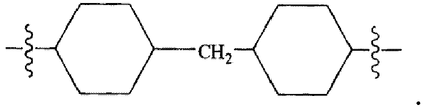

## Patentansprüche

1. Kosmetikzusammensetzung zum Schminken oder Pflegen keratinhaltiger Substanzen, die in einem physiologisch akzeptablen Medium mindestens ein Silikon-Polyurethan-Polymer und ein Silikonharz des Typs MQ umfasst,
wobei das Silikon-Polyurethan-Polymer das Reaktionsprodukt eines mit Hydroxylgruppen funktionalisierten Polyorganosiloxans, das der Struktur der Formel Ib entspricht: 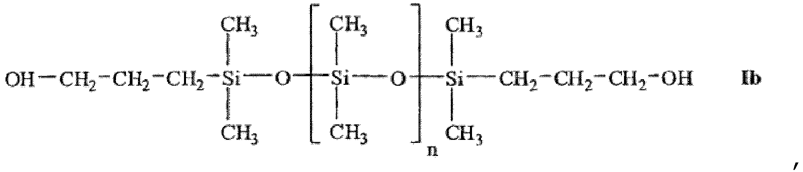
worin n eine ganze Zahl zwischen 0 und 5000, vorzugsweise zwischen 1 und 200, stärker bevorzugt zwischen 10 und 100 und noch stärker bevorzugt zwischen 10 und 50 ist,
mit einer Diisocyanatverbindung ist, die der Formel O=C=N-R¹-N=C=O entspricht, wobei R¹ eine divalente Kohlenwasserstoffgruppe ist, die 1 bis 20 Kohlenstoffatome enthält, und R¹ insbesondere eine Gruppe der Formel: 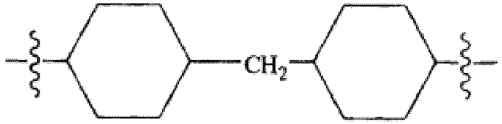 ist,
wobei das Silikonharz MQ vorzugsweise in fester Form, insbesondere in Pulverform vorliegt.

2. Zusammensetzung nach dem vorhergehenden Anspruch, **dadurch gekennzeichnet, dass** die Diisocyanatverbindung aus der aus 1,6-Hexamethylendiisocyanat, Methylendicyclohexyldiisocyanat, Isophorondiisocyanat und deren Kombinationen bestehenden Gruppe ausgewählt ist.

3. Zusammensetzung nach einem der vorhergehenden Ansprüche, **dadurch gekennzeichnet, dass** das Silikon-Polyurethan-Polymer in einem Gehalt im Bereich von 8 bis 16 Gew.-% Polymeraktivmasse, bezogen auf das Gesamtgewicht der Zusammensetzung, vorhanden ist.

4. Zusammensetzung nach einem der vorhergehenden Ansprüche, **dadurch gekennzeichnet, dass** das Silikonharz ein Harz des Typs MQ Alkylsiloxysilikate der Formel [(Rl)₃Si_{1/2}]ₓ(SiO_{4/2})_{y} ist, worin x und y ganze Zahlen im Bereich von 50 bis 80 sind und die Gruppe Rl einen Kohlenwasserstoffrest mit 1 bis 10 Kohlenstoffatomen, eine Phenylgruppe, eine Phenylalkylgruppe oder auch eine Hydroxylgruppe darstellt.

5. Zusammensetzung nach dem vorhergehenden Anspruch, **dadurch gekennzeichnet, dass** das Harz MQ des Trimethylsiloxysilikat-Typs ist.

6. Zusammensetzung nach einem der vorhergehenden Ansprüche, **dadurch gekennzeichnet, dass** das Silikonharz MQ in einem Gehalt im Bereich von 1 bis 20 Gew.-% Harz, bezogen auf das Gesamtgewicht der Zusammensetzung, vorzugsweise von 5 bis 10 Gew.-%, vorhanden ist.

7. Zusammensetzung nach einem der vorhergehenden Ansprüche, **dadurch gekennzeichnet, dass** das physiologisch akzeptable Medium mindestens ein flüchtiges oder nicht flüchtiges Kohlenwasserstofföl umfasst.

8. Zusammensetzung nach dem vorhergehenden Anspruch, **dadurch gekennzeichnet, dass** das flüchtige Kohlenwasserstofföl 8 bis 16 Kohlenstoffatome umfasst und vorzugsweise Isododecan ist.

9. Zusammensetzung nach einem der vorhergehenden Ansprüche, **dadurch gekennzeichnet, dass** sie mindestens ein Wachs und/oder mindestens ein lipophiles Geliermittel umfasst.

10. Zusammensetzung nach dem vorhergehenden Anspruch, **dadurch gekennzeichnet, dass** das Wachs ein Polyethylenwachs ist und das lipophile Geliermittel ein Ton ist.

11. Zusammensetzung nach einem der vorhergehenden Ansprüche, **dadurch gekennzeichnet, dass** sie mindestens einen aus Pigmenten, Perlmuttern, fettlöslichen Färbemitteln, Lacken (organisches Pigment) und deren Gemischen ausgewählten Farbstoff umfasst.

12. Verfahren zum Schminken oder Pflegen keratinhaltiger Substanzen, umfassend das Aufbringen mindestens einer Zusammensetzung nach einem der Ansprüche 1 bis 11 auf die keratinhaltigen Substanzen.

13. Verfahren zum Schminken oder Pflegen keratinhaltiger Substanzen, umfassend:
- das Aufbringen mindestens einer Zusammensetzung nach einem der Ansprüche 1 bis 11 auf die keratinhaltigen Substanzen als Basiszusammensetzung und
- das Aufbringen einer Überzugszusammensetzung auf die Basiszusammensetzung.

14. Verfahren nach Anspruch 13, **dadurch gekennzeichnet, dass** die Überzugszusammensetzung mit der Basiszusammensetzung inkompatibel ist.

15. Verwendung eines Silikonharzes MQ des Trimethylsiloxysilikat-Typs zum Verbessern der Abriebbeständigkeit und der Beibehaltung einer Kosmetikzusammensetzung, die in einem physiologisch akzeptablen Medium mindestens ein Silikon-Polyurethan-Polymer umfasst, wobei das Silikon-Polyurethan-Polymer das Reaktionsprodukt eines mit Hydroxylgruppen funktionalisierten Polyorganosiloxans, das der Struktur der Formel Ib entspricht: 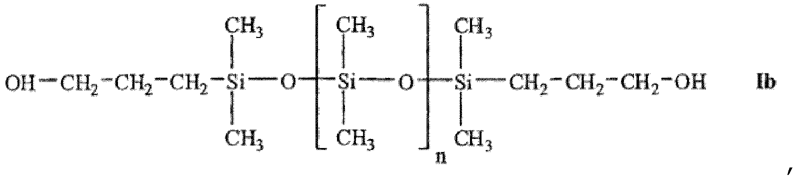
worin n eine ganze Zahl zwischen 0 und 5000, vorzugsweise zwischen 1 und 200, stärker bevorzugt zwischen 10 und 100 und noch stärker bevorzugt zwischen 10 und 50 ist,
mit einer Diisocyanatverbindung ist, die der Formel O=C=N-R¹-N=C=O entspricht, wobei R¹ eine divalente Kohlenwasserstoffgruppe ist, die 1 bis 20 Kohlenstoffatomen enthält, und R¹ insbesondere eine Gruppe der Formel: 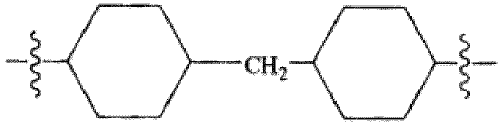 ist.

## Claims

1. Cosmetic composition for making up or caring for keratin materials comprising, in a physiologically acceptable medium, at least one silicone-polyurethane polymer and one silicone resin of MQ type,
said silicone-polyurethane polymer being the product of reaction of a polyorganosiloxane functionalized with hydroxyl groups corresponding to the structure of formula Ib: 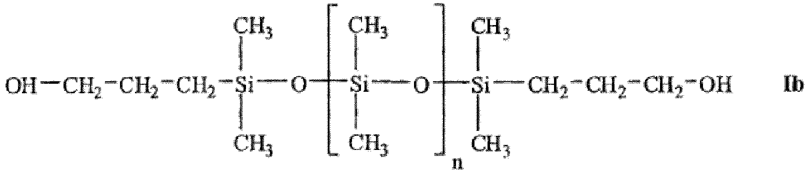
in which n is an integer between 0 and 5000, preferably between 1 and 200, more preferentially between 10 and 100, and more preferentially still between 10 and 50,
with a diisocyanate compound corresponding to the formula O=C=N-R¹-N=C=O, where R¹ is a divalent hydrocarbon group containing from 1 to 20 carbon atoms, and in particular R¹ is a group of formula: 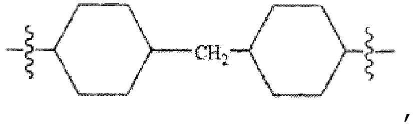
said MQ silicone resin preferably being in solid form, in particular in powder form.

2. Composition according to the preceding claim, **characterized in that** the diisocyanate compound is chosen from the group consisting of 1,6-hexamethylene diisocyanate, methylene dicyclohexyl diisocyanate, isophorone diisocyanate, and combinations thereof.

3. Composition according to either one of the preceding claims, **characterized in that** the silicone-polyurethane polymer is present at a content ranging from 8% to 16% by weight of polymer active material, relative to the total weight of the composition, preferably from 10% to 15% by weight.

4. Composition according to any one of the preceding claims, **characterized in that** the silicone resin is a resin of MQ alkylsiloxysilicates type of formula [(R1)₃Si_{1/2}]ₓ(SiO_{4/2})_{y} in which x and y are integers ranging from 50 to 80, and the R1 group represents a hydrocarbon radical having from 1 to 10 carbon atoms, a phenyl group, a phenylalkyl group or else a hydroxyl group.

5. Composition according to the preceding claim, **characterized in that** the MQ resin is of trimethylsiloxysilicate type.

6. Composition according to any one of the preceding claims, **characterized in that** the MQ silicone resin is present at a content ranging from 1% to 20% by weight of resin, relative to the total weight of the composition, preferably from 5% to 10% by weight.

7. Composition according to any one of the preceding claims, **characterized in that** the physiologically acceptable medium comprises at least one volatile or non-volatile hydrocarbon-based oil.

8. Composition according to the preceding claim, **characterized in that** the volatile hydrocarbon-based oil comprises from 8 to 16 carbon atoms, and is preferably isododecane.

9. Composition according to any one of the preceding claims, **characterized in that** it comprises at least one wax and/or at least one lipophilic gelling agent.

10. Composition according to the preceding claim, **characterized in that** the wax is a polyethylene wax and the lipophilic gelling agent is a clay.

11. Composition according to any one of the preceding claims, **characterized in that** it comprises at least one colourant chosen from pigments, nacres, liposoluble dyes, lakes (organic pigment) and mixtures thereof.

12. Process for making up or caring for keratin materials comprising the application to said keratin materials of at least one composition according to one of Claims 1 to 11.

13. Process for making up or caring for keratin materials comprising:
- the application to said keratin materials of at least one composition according to one of Claims 1 to 11, as base composition, and
- the application to said base composition of a coating composition.

14. Process according to Claim 13, **characterized in that** the coating composition is incompatible with the base composition.

15. Use of an MQ silicone resin of trimethylsiloxysilicate type, to improve the resistance to friction and the staying power of a cosmetic composition comprising, in a physiologically acceptable medium, at least one silicone-polyurethane polymer, said silicone-polyurethane polymer being the product of reaction of a polyorganosiloxane functionalized with hydroxyl groups corresponding to the structure of formula Ib: 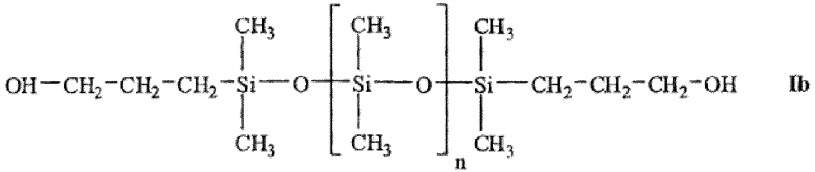
in which n is an integer between 0 and 5000, preferably between 1 and 200, more preferentially between 10 and 100, and more preferentially still between 10 and 50,
with a diisocyanate compound corresponding to the formula O=C=N-R¹-N=C=O, where R¹ is a divalent hydrocarbon group containing from 1 to 20 carbon atoms, and in particular R¹ is a group of formula:
